(19) Europäisches Patentamt
European Patent Office
Office européen des brevets

(11) Publication number: **0 040 507**
**B1**

(12) **EUROPEAN PATENT SPECIFICATION**

(45) Date of publication of patent specification: **21.09.83**

(51) Int. Cl.³: **C 07 D 461/00, A 61 K 31/475**

(21) Application number: **81302119.3**

(22) Date of filing: **13.05.81**

(54) **New vincamine derivatives, their production and pharmaceutical compositions containing them.**

(30) Priority: **16.05.80 JP 65774/80**

(43) Date of publication of application:
**25.11.81 Bulletin 81/47**

(45) Publication of the grant of the patent:
**21.09.83 Bulletin 83/38**

(84) Designated Contracting States:
**AT BE CH DE FR GB IT LI LU NL SE**

(56) References cited:
**GB - A - 1 405 127**
**GB - A - 1 445 956**
**GB - A - 1 518 987**

(73) Proprietor: **NIPPON ZOKI PHARMACEUTICAL CO. LTD.**
**10, 2-chome Hirano-machi Higashi-ku**
**Osaka (JP)**

(72) Inventor: **Toyomaki, Yoshio**
**30-501, 1, 3-chome Kataokadai**
**Kanmaki-cho Kitakatsuragi-gun Nara (JP)**

(74) Representative: **Crampton, Keith John Allen et al,**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 040 507 B1

# New vincamine derivatives, their production and pharmaceutical compositions containing them

The present invention is concerned with vincamine derivatives, a process for producing them and pharmaceutical compositions containing one or more of them as active ingredient.

Vincamine, one of the alkaloids from vinca minor L., has been known to have a vasodilative effect in cerebral vessels, and many derivatives of it have been synthesized so far. Vincamine is represented by the formula:

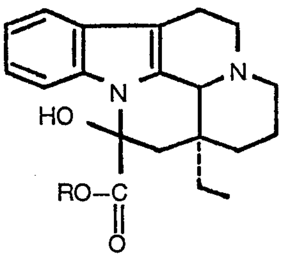

when R is a methyl radical, and vincaminic acid by the same formula when R is a hydrogen atom. When vincamine is administered to human beings orally, intravenously or intramuscularly, it is hydrolysed, mainly by liver esterase, to vincaminic acid, which lacks activity, and is excreted in the urine. Esters of vincaminic acid so far reported e.g. in UK Patent No GB—A—1405127 are those with various alcohols, which esters are readily hydrolysed by liver esterase. UK Patent No GB—A—1445956 discloses various esters of 16,17 dihydroapovincaminic acid. These two Patents also disclose pharmaceutical preparations containing the said esters and their therapeutic uses.

The present invention results from research aimed at obtaining vincamine derivatives that are not easily inactivated *in vivo* and therefore might be expected to retain the pharmacological effects of vincamine, viz. in regulating the circulation of blood in, and the metabolism if, the brain. This research has resulted in the discovery that new vincaminic acid thio-esters have similar or superior activity to vincamine and are hardly hydrolysed at all by liver esterase.

The present invention provides thio-esters of vincaminic acid or deoxyvincaminic acid and their pharmacologically acceptable salts, which are useful as cerebral metabolic and circular regulators having a prominent retaining effect. The compounds according to the present invention have the following general formula (I):

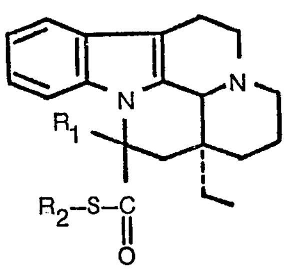

(I)

in which $R^1$ is a hydrogen atom or a hydroxy group and $R_2$ is a $C_{1-4}$ alkyl group, or are their pharmacologically acceptable salts.

The alkyl group $R_2$ may be linear or branched, specifically, methyl, ethyl, propyl, isopropyl, utyl, isobutyl, *sec*-butyl or *t*-butyl.

The compounds according to the present invention include the individual optically active substances and all mixtures of both epimers. (The 14-position is asymmetric).

The pharmacologically acceptable salts of the compound (I) are acid-addition salts with inorganic or organic acids, such as hydrochloric acid, hydrobromic acid, sulphuric acid, phosphoric acid, formic acid, acetic acid, citric acid, tartaric acid, lactic acid, glycolic acid, malic acid, malonic acid, maleic acid, gluconic acid, succinic acid, stearic acid, ascorbic acid, phthalic acid, benzoic acid, nicotinic acid, salicylic acid, sulphosalicylic acid, palmitic acid, mandelic acid, methanesulphonic acid, benzenesulphonic acid, *p*-toluenesulphonic acid and 2-hydroxy-3-naphthoic acids; or are quaternary salts with halides such as methyl, ethyl, propyl, butyl or benzyl chloride, bromide or iodide.

In accordance with the present invention, the novel compounds are produced by reacting vincaminic acid or deoxyvincaminic acid, which has the general formula (II), including its epimers and mixtures of epimers, or a reactive derivative, such as an acid halide, of such a compound,

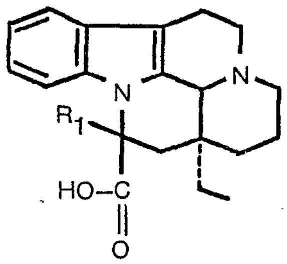

(II)

with an alkanethiol of the general formulr $R_2$—SH, where $R_1$ and $R_2$ are as defined above. The above reaction may be carried out in the presence of base, such as triethylamine or pyridine, and preferably an added synthetic reagent, such as diphenylphosphorylazide. In this reaction, an inactive solvent such as dimethylformamide, acetone, tetrahydrofuran, dioxane, dimethyl sulphoxide or chloroform can be used. Cooled conditions or mild conditions involving ambient temperature may be preferable.

After the reaction, the desired compound can be isolated and purified by usual means such as extraction, chromatography, recrystallization and/or reprecipitation. Furthermore, if desired, conversion to an acid-addition salt or quaternary salt can be carried out by a known method.

The following examples illustrate but do not limit the process for producing compounds of the present invention.

Example 1

2.5 ml of triethylamine and 2.53 ml of diphenylphosphoryl-azide were mixed with 250 ml of dimethylformamide. 2 g of vincaminic acid was suspended in the resulting mixture and 7.2 ml of ethanethiol was added dropwise. The mixture was reacted at about 20°C for 24 hours. After the unreacted vincaminic acid had been filtered off, water was added to the filtrate, which was then allowed to stand overnight. The white precipitate thus generated was filtered and dissolved in chloroform. The solution was washed with water and dried over anhydrous sodium sulphate. The solvent was distilled off under reduced pressure and column chromatography using silica gel then carried out. After elution with chloroform, concentration, and drying the fractions containing the desired compound, recrystallization from ethanol gave vincaminic acid thioethyl ester in the form of a colourless granular crystalline material.

m.p.: 203—203.5°C (Dec.)

I R: absorption attributed to thio-ester at 1685 cm-1

Elementary analysis ($C_{22}H_{28}N_2O_2S = 384.5$);

| | C% | H% | N% | S% |
|---|---|---|---|---|
| Calculated: | 68.72 | 7.34 | 7.28 | 8.34 |
| Found: | 68.80 | 7.50 | 7.11 | 8.32 |

Example 2

1.0 g of deoxyvincaminic acid, 1.2 ml of diphenylphosphorylazide, 2.0 ml of ethanethiol and 1.2 ml of triethylamine were added to 30 ml of dimethylformamide under cooling with ice. The mixture was stirred for 3 hours at room temperature, and the reaction continued till thin-layer chromatography confirmed that no reactant remained. After addition of cold water, followed by standing, the resulting white suspended product was filtered off, washed with water, dissolved in chloroform, washed with a saturated aqueous solution of sodium chloride and dried over anhydrous sodium sulphate. Thereafter, in the same manner as described in Example 1, deoxyvincaminic acid thioethyl ester was obtained in the form of colourless acicular crystals.

m.p.: 140—141°C (Dec.)

I R: absorption attributed to thio-ester at 1675 $cm^{-1}$

Elementary analysis ($C_{22}H_{28}N_2OS = 368.55$);

| | C% | H% | N% | S% |
|---|---|---|---|---|
| Calculated: | 71.70 | 7.66 | 7.60 | 8.70 |
| Found: | 71.40 | 7.63 | 7.50 | 8.91 |

The pharmacological tests of the compounds according to the present invention are shown below.

(1) Anti-hypobare hypoxia test

5 mg/Kg of a compound of the present invention or a compound used for comparison was administered intravenously to ddy-strain male mice at tail. After ten minutes, the mice were placed in vacuum desiccator containing at the bottom a petri dish with a saturated aqueous solution of potassium hydroxide immersing a thimble. The pressure in the desiccator was reduced to 24 kPa. Thereafter, the survival time of the mice was measured at every five seconds. The operation was carried

out twenty times.

Average time ± standard error was determined, regarding 600 seconds as the case of survival time over 10 minutes.

The results are shown below.

| Tested Compounds | Survival time (sec.) |
|---|---|
| control | 98.0 ± 14.6 |
| vincamine | 149.0 ± 29.6 |
| vincaminic acid ethyl ester | 114.5 ± 18.4 |
| vincaminic acid thioethyl ester | 139.3 ± 20.4 |

The experiment was repeated but with 15 mg/Kg intraperitoneal administration. The results are shown below.

| Tested compounds | Survival time (sec.) |
|---|---|
| control | 75.8 ± 6.95 |
| vincamine | 270.0 ± 57.96 |
| deoxyvincaminic acid thioethyl ester | 350.8 ± 71.78 |
| pentoxifylline (75 mg/Kg) | 80.8 ± 6.50 |

(2) Hydrolysis by liver homogenate

The rate of hydrolysis of the compounds of the present invention and the compounds used for comparison was determined using liver homogenate solution of guinea pig. The tested compounds were added to 0.5% (by weight) homogenate solution to a final concentration of 25 μg/ml and incubated at 30°C. At every incubation time the amount of product, i.e. vincaminic acid or deoxyvincaminic acid, was measured by hydrolysis. The results are shown in Fig. 1 and Fig. 2 of the accompanying drawings, which show the rate of hydrolysis of the compounds of the present invention and the compounds used for comparison by liver homogenate of guinea pig.

(3) Acute toxicity

$LD_{50}$ of the compound of the present invention, deoxyvincaminic acid thioethyl ester, using male mice, is 170 mg/Kg in case of intraperitoneal administration and more than 3,000 mg/Kg in case of oral administration.

As shown by the results of the above, compounds according to the present invention have similar or superior effect on extending the survival time compared with vincamine or its corresponding alcohol esters, and are hardly hydrolysed at all by liver esterase. Therefore, these compounds are useful as long-acting cerebral metabolic and circulation regulators for treatment of various diseases caused by disorder of the cerebral blood flow and/or depression of oxygenation in the brain, for example, cerebral apoplexy (cerebral haemorrhage, cerebral thrombosis, cerebral embolism), cerebral arteriosclerosis, hypertensive disturbance of cerebral circulation, post-traumatic cerebral symptoms, defects of memory, headache, deafness, tinnitus, vertigo, Menière's disease, and retinopathy. Particularly, a lower dosage or less frequent administration may be possible than in the case of the known compounds.

As medicines, the compounds of the present invention can be prepared in combination with suitable pharmaceutical carriers or diluents, and can be prescribed by conventional methods for oral or non-oral dosage forms. The compounds of the present invention can be used in the form of their pharmacologically acceptable salts. They may be used singly or in an appropriate combination with other pharmaceutically active components.

For oral administration, the compounds can be prepared, for example, as tablets, pills, capsules, powders, granules and the like using at least one vehicle such as starch, lactose, sucrose, mannitol or carboxymethylcellulose. This pharmaceutical preparation may also contain other vehicles such as a lubricant, for example, magnesium stearate, sodium lauryl sulphate or talc, a binder, for example, dextrin, crystalline cellulose, polyvinylpyrrolidone, acacia gum, corn starch or gelatine, and a disintegrator, for example, potato starch or carboxmethylcellulose, as occasion demands. Further, the

compounds can be administered in the form of suspensions, emulsions, syrups and elixirs, which may contain a flavour or colouring agent.

As non-oral preparations, the compounds can be administered in sterilized aqueous or nonaqueous injectable solutions or suspensions. Diluents used include distilled water for injection, physiological saline solution, aqueous solution of dextrose, vegetable oil for injection, propylene glycol, and polyethylene glycol. An agent to render the injection isotonic, a solubilizer, a stabilizer, an antiseptic and/or a pain-removing agent may be also contained in this preparation, if necessary. Further, this type of preparation can be made in the form of a sterilized solid composition to be dissolved in sterilized water or other sterilized medium for injection.

As other non-oral preparations, suppositories can be prepared by mixing the compound with suitable bases.

The desired dosage of the compound of the present invention is variable according *inter alia* to subject, route of administration, and period of treatment. Generally, for an adjult, administration of 1—500 mg per day may be preferable to obtain the desired effect, and, for example, one to several units containing 1—100 mg of the compound of the present invention can be administered per day.

Illustrating and non-limiting examples of pharmaceutical compositions containing as active ingredient a compound of the present invention are shown below.

Prescription example 1. (tablet containing 25 mg of the compound of the present invention)

| Component | Amount per tablet (mg) |
|---|---|
| compound of the invention | 25 |
| lactose | 120 |
| corn starch | 50 |
| magnesium stearate | 5 |
| total | 200 mg |

The compound of the invention, lactose and corn starch are equally mixed, kneaded together with water and shaped into granules by a granulating machine. After drying by warm air, the granules are mixed with magnesium stearate and shaped into tablets by a tablet tableting machine.

Prescription example 2. (capsule containing 30 mg of the compound of the invention)

| Component | Amount per capsule (mg) |
|---|---|
| compound of the invention | 30 |
| lactose | 120 |
| total | 150 mg |

The above components are equally mixed and charged into hard capsules.

Prescription example 3. (injection of 3 ml containing 15 mg of the compound of the invention)

| Component | Ampount in 3 ml (mg) |
|---|---|
| compound of the invention | 15 |
| sodium chloride | proper amount |
| distilled water for injection | proper amount |
| total | 3 ml |

The above components are mixed to form a uniform solution. The solution is filtered and charged in 3 ml ampoule. The ampoule is closed by fusion, then sterilized.

Prescription example 4. (suppository containing 50 mg of the compound of the invention)

| Component | | Amount per one unit (mg) |
|---|---|---|
| compound of the invention | | 50 |
| cacao butter | | 1950 |
| | total | 2000 mg |

The compound of the invention is added to the molten base and mixed thoroughly. Stirring is continued till shaping is achieved.

**Claims**

1. Compounds of the general formula (I):

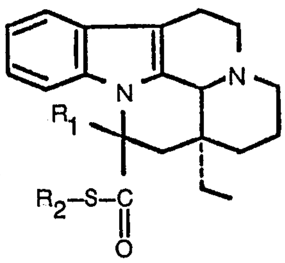

(I)

in which $R_1$ is a hydrogen atom or a hydroxy group and $R_2$ is a $C_{1-4}$ alkyl group, and compounds that are their pharmacologically acceptable salts.

2. Vincaminic acid thioethyl ester.

3. Deoxyvincaminic acid thioethyl ester.

4. A method of producing a compound as claimed in Claim 1 comprising reacting a compound of the general formula (II):

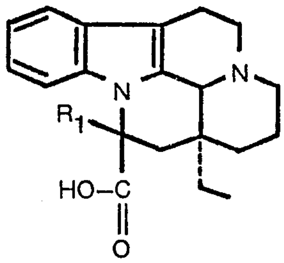

(II)

or an active derivative of such a compound with a compound of the general formula $R_2$—SH where $R_1$ and $R_2$ are as defined in Claim 1, and optionally converting the resulting compound to a pharmacologically acceptable salt.

5. A method as claimed in Claim 4 carried out in the presence of base.

6. A method as claimed in Claim 4 or 5 carried out in the presence of diphenylphosphorylazide.

7. Pharmaceutical composition for use as a cerebral metabolic and circulatory regulator and containing as an active ingredient a compound as claimed in Claim 1.

8. A composition as claimed in Claim 7 in which the active ingredient is vincaminic acid thioethyl ester or a pharmacologically acceptable salt thereof.

9. A composition as claimed in Claim 7 in which the active ingredient is deoxyvincaminic acid thioethyl ester or a pharmacologically acceptable salt thereof.

10. A compound as claimed in Claim 1, 2 or 3, for use as a cerebral metabolic and circulatory regulator.

**Patentansprüche**

1. Verbindungen der allgemeinen Formel (I):

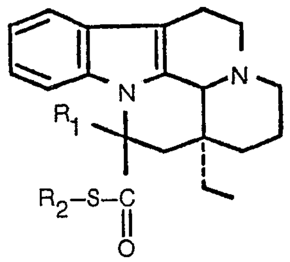

(I)

in der $R_1$ ein Wasserstoffatom oder eine Hydroxygruppe und $R_2$ eine $C_{1-4}$ Alkylgruppe ist, und Verbindungen, die ihre pharmakologisch annehmbaren Salze sind.

2. Vincaminsäurethioäthylester.

3. Deoxyvincaminsäurethioäthylester.

4. Verfahren zur Herstellung einer Verbindung nach Anspruch 1, bei dem eine Verbindung der allgemeinen Formel (II):

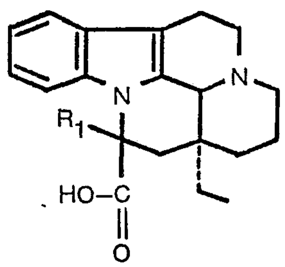

(II)

oder ein aktives Derivat einer solchen Verbindung mit einer Verbindung der allgemeinen Formel $R_2$—SH, worin $R_1$ und $R_2$ wie im Anspruch 1 definiert sind, zuf Reaktion gebracht wird und die entstehende Verbindung fakultativ in ein pharmakologisch annehmbares Salz umgewandelt wird.

5. Verfahren nach Anspruch 4 durchgeführt in Gegenwart einer Base.

6. Verfahren nach Anspruch 4 oder 5 durchgeführt in Gegenwart von Diphenylphosphorylazid.

7. Pharmazeutische Verbindung zur Verwendung als zerebralen Stoffwechsel- und Kreislaufregulator und enthaltend als einen aktiven Bestandteil eine Verbindung nach Anspruch 1.

8. Verbindung nach Anspruch 7, in der der aktiv Bestandteil Vincaminsäurethioäthylester oder eines seiner pharmakologisch annehmbaren Salze ist.

9. Verbindung nach Anspruch 7, in der der aktive Bestandteil Deoxyvincaminsäureethoxyäthylester oder eines seiner pharmakologisch annehmbaren Salze ist.

10. Verbindung nach Anspruch 1, 2 oder 3 zur Verwendung als zelebralen Stoffwechsel- und Kreislaufregulator.

**Revendications**

1. Composés de formule générale (I):

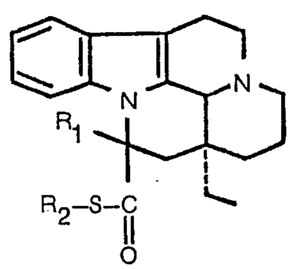

(I)

où $R_1$ est un atome d'hydrogène ou un groupe hydroxy et $R_2$ est un groupe alkyle en $C_{1-4}$, et les composés qui sont leurs sels acceptables en pharmacologie.

2. Ester thioéthylique de l'acide vincaminique.

3. Ester thioéthylique de l'acide désoxyvincaminique.

4. Un procédé pour produire un composé comme revendiqué dans la revendication 1, comprenant la réaction d'un composé de formule générale (II):

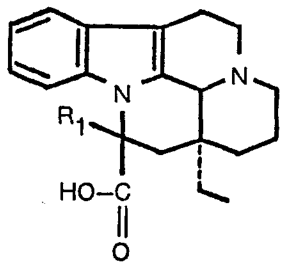

(II)

ou d'un dérivé actif d'un tel composé, avec un composé de formule générale $R_2$—SH où $R_1$ et $R_2$ sont comme défini dans le revendication 1, et éventuellement la conversion du composé obtenu en un sel acceptable en pharmacologie.

5. Un procédé comme revendiqué dans la revendication 4, mis en pratique en présence d'une base.

6. Un procédé comme revendiqué dans la revendication 4 ou 5, mis en pratique en présence de diphénylphosphorylazide.

7. Composition pharmaceutique pour l'emploi comme régulateur du métabolisme et de la circulation du cerveau et contenant comme ingrédient actif un composé comme revendiqué dans la revendication 1.

8. Une composition comme revendiqué dans la revendication 7, dans laquelle l'ingrédient actif est l'ester thioéthylique de l'acide vincaminique ou un de ses sels acceptavles en pharmacologie.

9. Une composition comme revendiqué dans la revendication 7, dans laquelle l'ingrédient actif est l'ester thioéthylique de l'acide désoxyvincaminique ou un de ses sels acceptables en pharmacologie.

10. Un composé comme revendiqué dans la revendication 1, 2 ou 3, pour l'emploi comme régulateur du métabolisme et de la circulation du cerveau.

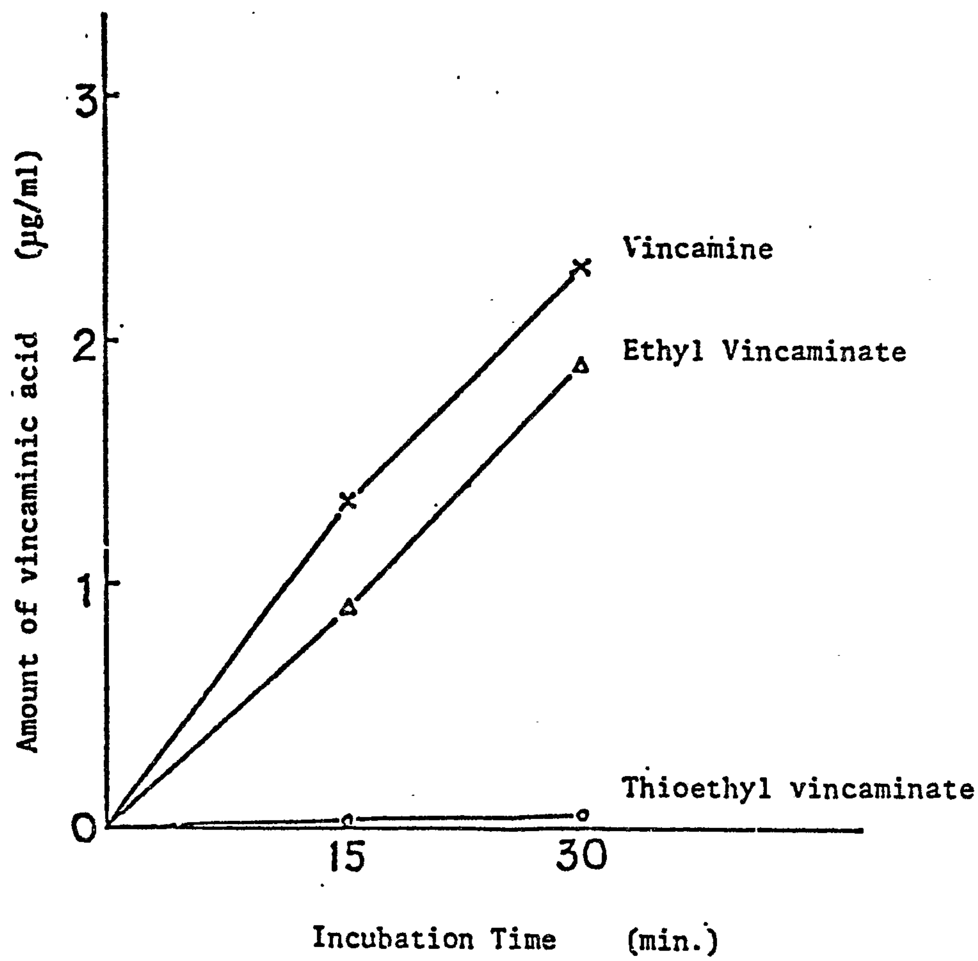
Amount of vincaminic acid (µg/ml)
3
2
1
0
Vincamine
Ethyl Vincaminate
Thioethyl vincaminate
15
30
Incubation Time (min.)

Fig. 1

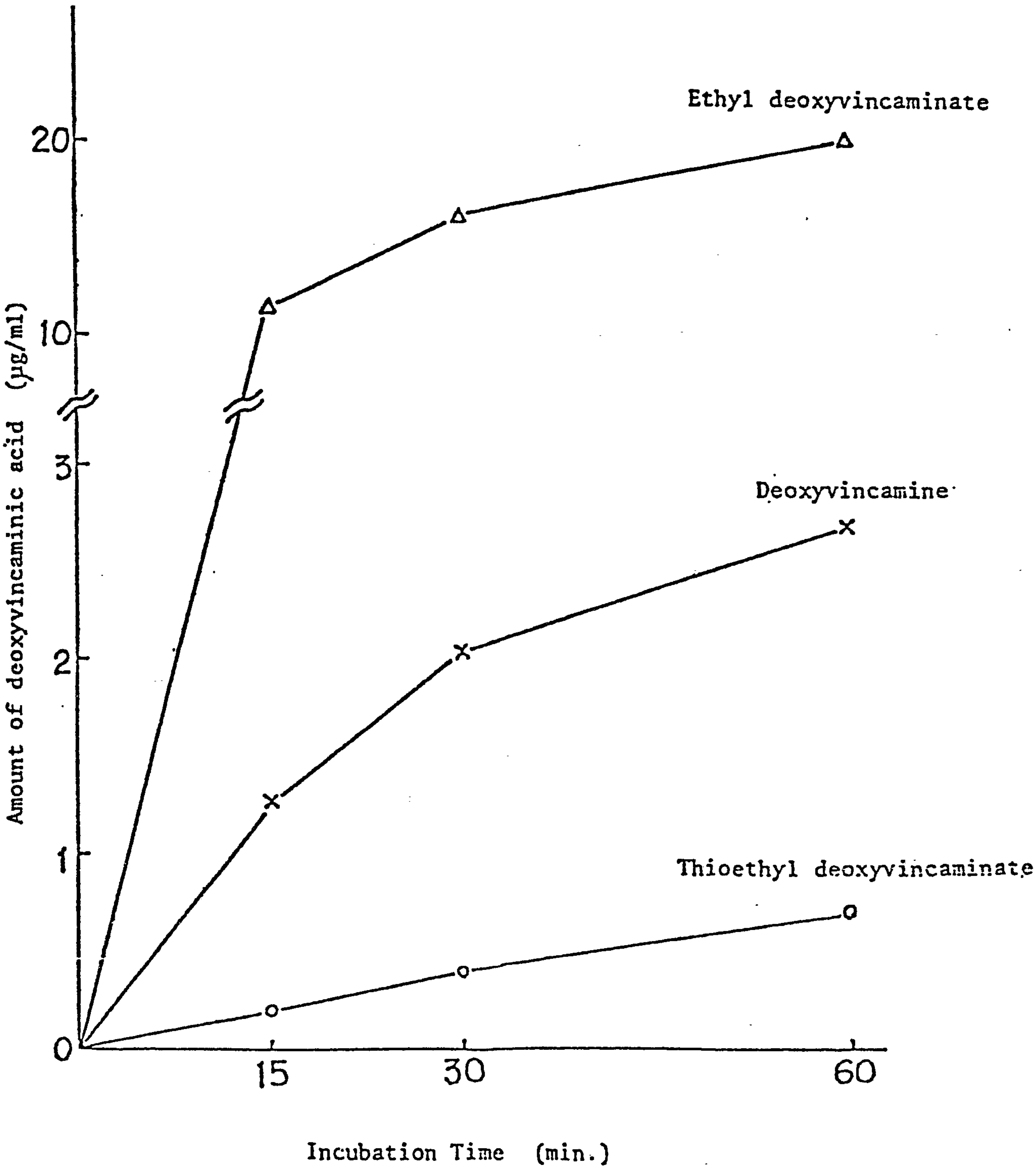

Ethyl deoxyvincaminate
Deoxyvincamine
Thioethyl deoxyvincaminate
Amount of deoxyvincaminic acid (μg/ml)
20
10
3
2
1
0
15
30
60
Incubation Time (min.)


Fig. 2